# EUROPÄISCHE PATENTSCHRIFT

(11) **EP 0 753 498 B1**
(45) Veröffentlichungstag und Bekanntmachung des Hinweises auf die Patenterteilung: **13.10.1999**
(21) Anmeldenummer: 96110965.9
(22) Anmeldetag: 08.07.1996
(51) Int. Cl.: C07C 2/66, C07C 15/16

(54) **Verfahren zur Herstellung von Diarylethanen**
Process for the preparation of diarylethanes
Procédé de préparation de diaryléthanes

(30) Priorität: 14.07.1995 DE 19525682
(43) Veröffentlichungstag der Anmeldung: 15.01.1997
(73) Patentinhaber: BASF AKTIENGESELLSCHAFT, 67056 Ludwigshafen (DE)
(72) Erfinder: Gehrer, Eugen, Dr., 67069 Ludwigshafen (DE); Massonne Klemens, Dr., 67368 Westheim (DE); Harder, Wolfgang, Dr., 69469 Weinheim (DE)

(56) Entgegenhaltungen:
- EP-A- 0 317 907
- FR-A- 1 281 757
- GB-A- 1 579 815
- GB-A- 2 021 637
- US-A- 4 289 918
- US-A- 4 689 436

## Beschreibung

Die vorliegende Erfindung betrifft eine Verfahren zur Herstellung von Diarylethanen durch Umsetzung von Aromaten mit Styrolen in Gegenwart eines sauren Heterogenkatalysators bei erhöhten Temperaturen und erhöhten Drücken.

Die Herstellung von Diphenylethan durch Umsetzung von Aromaten mit Styrol(-derivaten) ist in JP63-238028 (1988) unter Verwendung von Y-Zeolithen, in Polymer Journal, Vol. 12, No. 6, Seite 407 (1980) unter Verwendung von Nafion, Amberlyst 15 oder CF₃SO₃H, in Gegenwart von amorphen Siliziumaluminaten (GB-A 1579815, GB-A 2021637) von Perfluorpolymeren (US-A 4 289 918) bzw. von Heteropolysäuren (US-A 4 689 436) sowie in EP-A-421 340 unter Verwendung von L-Zeolithen beschrieben. Die beschriebenen Synthese liefern nur beim Einsatz von alkylierten Aromaten brauchbare Ausbeuten, die jedoch zu wünschen übrig lassen. Bei der Verwendung von Benzol als Aromat entstanden überwiegend Oligomere des Styrols.

Der vorliegenden Erfindung lag daher die Aufgabe zugrunde, den zuvor genannten Nachteilen abzuhelfen.

Demgemäß wurde ein neues und verbessertes Verfahren zur Herstellung von Diarylethanen der allgemeinen Formel I 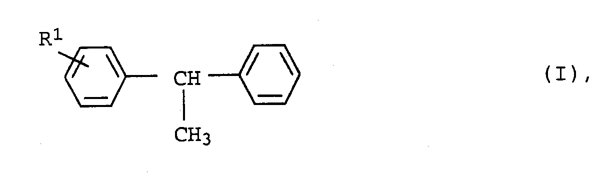 in der R¹ Wasserstoff oder C₁- bis C₈- Alkyl bedeutet, durch Umsetzung von Benzol mit Styrolen der allgemeinen Formel III 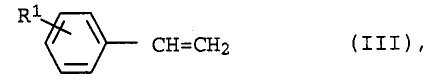 in der R¹ die oben genannten Bedeutungen hat, bei Temperaturen von 150 bis 350°C und Drücken von 5 bis 200 bar in Gegenwart von sauren Heterogenkatalysatoren mit der Maßgabe, daß der Druck so hoch ist, daß unter den Reaktionsbedingungen das System in flüssiger oder überkritischer Phase bleibt, gefunden, wobei man als saure Heterogenkatalysatoren großporige Zeolithe einsetzt.

Das erfindungsgemäße Verfahren läßt sich wie folgt durchführen:

Die Umsetzung von Benzol mit den Styrolen III kann diskontinuierlich oder bevorzugt kontinuierlich in Druckapparaturen wie Autoklaven in flüssiger oder überkritischer Phase bei Temperaturen von 150 bis 350°C, bevorzugt 180 bis 230°C, besonders bevorzugt 190 bis 220°C und Drücken von 5 bis 200 bar, bevorzugt 10 bis 130 bar, besonders bevorzugt unter Eigendruck oder durch zusätzliches Aufpressen von 1 bis 50 bar in Gegenwart eines sauren Heterogenkatalysatorsdurchgeführt werden. Der Druck muß also so hoch sein, daß unter den Reaktionsbedingungen das System flüssig oder gegebenenfalls in der überkritischen Phase bleibt.

Das erfindungsgemäße Verfahren kann beispielsweise in einem kontinuierlich gerührten Reaktors oder ein Festbettreaktor durchgeführt werden.

Bei der Umsetzung in einem Festbettreaktor wird in der Regel der saure Heterogenkatalysator in Form von Formkörpern in dem Festbettreaktor vorlegt und das Reaktionsgemisch unter starker Rückdurchmischung mehrmals durch dieses Festbett gefahren, wobei dem Kreisstrom kontinuierlich frische Eduktlösung zudosiert und eine äquivalente Menge Produktlösung kontinuierlich abgezogen wird.

Als saure Heterogenkatalysatoren eignen sich in der Regel stark saure, großporige Zeolithe wie 12-Ring-Zeolithe vom Typ Beta-Zeolith (BEA), HY-Zeolith oder Mordenite. Der Katalysator kann in der Regel entweder in Pulverform im Reaktionssystem suspendiert werden oder als Formkörper im einem Festbettreaktor eingesetzt werden.

Beta-Zeolithe sind beispielsweise aus der US-A-3 308 069 bekannt. Sie lassen sich mittels Tetraethylammoniumhydroxid bei Temperaturen im Bereich von 100 bis 150°C aus Gelen mit der Zusammensetzung TEA₂O:SiO₂:Al₂O₃:Na₂O:H₂O kristallisieren, wobei das SiO₂/Al₂O₃-Verhältnis im Bereich von 10:1 bis 200:1, das Verhältnis Na₂O/TEAOH von 0 bis 1:1, TEAOH/SiO₂ von 0,1:1 zu 1:1 und H₂O/TEAOH von 20:1 bis 75:1 liegen kann. Sie besitzen ein großporiges, dreidimensionales Porensystem mit 12-gliederigen Ringen mit Durchmessern von 6,5 x 5,6 und 7,5 x 5,7 Å.

Die Reaktion wird in der Regel bevorzugt unter Bedingungen durchgeführt, die nur eine geringe Konzentration von Styrol zulassen. Dies kann durch den Einsatz von verdünnten Styrol-Lösungen oder durch das langsame Zudosierung des Styrols zum Reaktionssystem erfolgen. Eine besonders bevorzugte Methode zur diskontinuierlichen Herstellung besteht darin, daß man den Aromaten zusammen mit dem darin suspendierten Katalysator im einem Rührkessel vorlegt und das Styrol (oder eine Styrol-Lösung) während der Reaktion so langsam zutropft, daß sich ein Fließgleichgewicht zwischen dem zudosierten und dem abreagierenden Styrol auf einem niedrigen Konzentrationsniveau einstellt. Die stationäre Konzentration an Styrol sollte 3%, bevorzugt 0,1%, besonders bevorzugt 0,05% nicht überschreiten.

Die Diphenylethane I können über den Sumpf einer Destillationskolonne erhalten werden.

Die Substituenten R¹, R², R³, R⁴, R⁵ und R⁶ in den Verbindungen I, II und III haben folgende Bedeutungen und R¹ in ortho-, meta- oder para-Stellung stehen kann:
- Wasserstoff,
- C₁- bis C₈-Alkyl, bevorzugt C₁- bis C₄-Alkyl wie Methyl, Ethyl, n-Propyl, iso-Propyl, n-Butyl, iso-Butyl, sec.-Butyl, tert.- Butyl, besonders bevorzugt Methyl und Ethyl.

Die Verbindungen I, II und III, in denen die Substituenten R¹, R², R³, R⁴, R⁵ und R⁶ gleichzeitig Wasserstoff bedeuten, 1,1-Diphenylethan, Benzol und Styrol, sind besonders bevorzugt.

Diphenylethan ist ein wichtiges Zwischenprodukt für die Herstellung von Diphenylethen, welches als Comonomeres für die Herstellung von Kunststoffen verwendet wird. Diphenylethen kann durch katalytische Dehydrierung von Diphenylethan in guten Ausbeuten gewonnen werden.

### Beispiele

Zur Analyse der nachfolgenden Experimente wurde ein Gaschromatograph mit einer 30 m langen Kapillarsäule (DB5, 0.1 µm) verwendet. (Temperaturprogramm: 5 Minuten bei 60°C, 10°C/Minute bis 300°C, 15 Minuten Endtemperatur). Die Quantifizierung erfolgte mit Hilfe Toluol als innerem Standard.

Die drei entstandenen Dimere (Identifizierung mittels GC/MS) wurden in der folgenden Zusammenstellung zusammengezählt. Ihr Eichfaktor wurde mit dem für Diphenylethan gleichgesetzt. Die Eichfaktoren für Benzol (nicht aufgelistet), Styrol und Diphenylethan wurden über eine Konzentrationsreihe mit Toluol als mit internem Standard bestimmt.

### Katalysator A

100 g β-Zeolith-Pulver (ZEOCAT® BETA der Firma Uetikon, SiO₂ = 91 %, Al₂O₃ = 7.8 %, Na₂O = 0.5 %, K₂O = 0.7%) wurden mit 1 Liter 20%iger NH₄Cl-Lösung 2 h bei 80°C gerührt, dekantiert, 3 mal mit je 500 ml Wasser gewaschen, bei 110°C 16 h getrocknet und bei 500°C 3 h calciniert.

### Beispiel 1

2 g Katalysator A wurden zusammen mit 30 g Benzol in einem 50 ml Glasdruckgefäß vorgelegt und unter Rühren wurde eine Mischung von 10 % Styrol und 90 % Benzol mit einer Rate von 10 ml/h zudosiert.

Über dem Glasdruckgefäß war eine Beruhigungszone in Form eines 20 cm langen Druckglasrohres (Durchmesser 1 cm) installiert, in der sich der Katalysator absetzen konnte. Der Innendruck des Gefäßes wurde durch ein Überströmventil bei 15 bar gehalten. Die Temperatur des Glasdruckautoklaven wurde auf 200°C gebracht. Der Produktaustrag wurde mittels GC analysiert und die Ergebnisse in Tabelle 1 zusammengefaßt.

**Tabelle 1**

| Laufzeit [h] | Styrol [%] | 1,1-Diphenylethan | Umsatz [%] | Ausbeute [%] |
|---|---|---|---|---|
| 6 | 0,2 | 7,08 | 98 | ---- |
| 22 | 0,13 | 12,7 | 98,7 | 72,6 |
| 90 | 0,06 | 16,7 | 99,4 | 95,4 |
| 150 | 0,05 | 13,5 | 99,5 | 76,9 |
| 200 | 0,03 | 14,2 | 99,7 | 81,3 |
| 310 | 0,03 | 16,9 | 99,7 | 96,6 |

### Beispiel 2

Ein zu Beispiel 1 analoger Versuch wurde unter Zusatz von 0.1 % t-Butylbrenzcatechin als Stabilisator gefahren. Die Ergebnisse sind in Tabelle 2 zusammengefaßt.

**Tabelle 2**

| Laufzeit [h] | Styrol [%] | 1,1-Diphenylethan | Umsatz [%] | Ausbeute [%] |
|---|---|---|---|---|
| 5 | 0,02 | 3,45 | 99,8 | ---- |
| 21 | <0,02 | 12,0 | 100 | 68,6 |
| 101 | 0,04 | 17,3 | 99,6 | 98,8 |
| 149 | 0,2 | 17,4 | 98 | 98,9 |
| 197 | 1,17 | 14,8 | 88 | 84,4 |

### Beispiel 3

Analog zu Beispiel 1 wurde in einem 250 ml Dreihalskolben 10 g Katalysator und 100 g Benzol vorgelegt. Nach dem Aufheizen auf 80°C und Atmosphärendruck wurde eine Mischung von 10 % Styrol und 90 % Benzol mit einer Rate von 10 ml/h zudosiert. Es zeigte sich eine rasche Desaktivierung bei diesen Bedingungen. Die Ergebnisse sind in Tabelle 3 zusammengefaßt.

**Tabelle 3**

| Laufzeit [h] | Styrol [%] | 1,1-Diphenylethan | Umsatz [%] | Ausbeute [%] |
|---|---|---|---|---|
| 6 | <0,01 | 2,86 | 100 | ---- |
| 22 | <0,01 | 13,5 | 100 | 77,0 |
| 26 | 0,06 | 17,3 | 99,4 | 98,8 |
| 30 | 0,28 | 13,7 | 97,2 | 78,2 |
| 38 | 1,04 | 9,5 | 89,6 | 54,3 |
| 54 | 4,14 | 3,24 | 58,6 | 18,5 |
| 90 | 8,74 | 0,34 | 12,6 | 1,9 |

### Katalysator B

220 g β-Zeolith-Pulver (ZEOCAT® BETA der Firma Uetikon, SiO₂ = 91 %, Al₂O₃ = 7.8 %, Na₂O = 0.5 %, K₂O = 0.7 %) wurden mit 5 % Walocel® und 230 g Wasser 45 Minuten im Kneter verdichtet. Anschließend wurde die Masse mit 70 bar Preßdruck zu 2 mm Strängen verformt, bei 110°C getrocknet und bei 500°C 16 h calciniert.

195 g dieser Stränge wurden mit 3 Liter 20%iger NH₄Cl-Lösung bei 80°C 2 h ausgetauscht und mit 10 Liter Wasser gewaschen. Danach wurde ein zweiter Austausch mit ebenfalls 3 Liter 20%iger NH₄Cl-Lösung bei 80°C 2 h vorgenommen, das Produkt in der Folge Cl-frei gewaschen, bei 110°C getrocknet und 5 h bei 500°C calciniert.

### Beispiel 4

In einem 50 ml Stahlautoklaven wurden in einem Katalysatorkorb 10 g β-Zeolithstränge (Katalysator B) vorgelegt. Der Autoklav wurde mit 30 ml Benzol gefüllt, unter Rühren auf 250°C geheizt und kontinuierlich eine Mischung von 10 % Styrol und 90 % Benzol zudosiert. Das Produktgemisch wurde kontinuierlich über ein Überströmventil abgezogen. Das Überströmventil hielt den Druck auf 50 bar. Die Produktausträge wurden mittels GC analysiert. Die Ergebnisse sind in Tabelle 4 zusammengefaßt.

**Tabelle 4**

| Laufzeit [h] | Temperatur [°C] | Belastung [g/h] | Styrol [%] | Diphenylethan [%] | Umsatz [%] | Ausbeute [%] |
|---|---|---|---|---|---|---|
| 24 | 250 | 50 | 0,02 | 8,3 | 99,8 | 47 |
| 48 | 230 | 50 | 0,03 | 12,7 | 99,7 | 72,6 |
| 72 | 230 | 50 | 0,03 | 14,7 | 99,7 | 84 |
| 96 | 230 | 50 | 0,03 | 13,5 | 99,7 | 77,3 |
| 120 | 230 | 50 | 0,04 | 14,1 | 99,6 | 80,6 |
| 144 | 230 | 50 | 0,04 | 14,2 | 99,6 | 81,1 |
| 168 | 230 | 100 | 0,09 | 14,7 | 99,1 | 84 |
| 192 | 230 | 200 | 0,14 | 15,8 | 98,6 | 90,2 |
| 212 | 230 | 200 | 1,78 | 11,1 | 82,2 | 63,4 |

## Patentansprüche

1. Verfahren zur Herstellung von Diarylethanen der allgemeinen Formel I 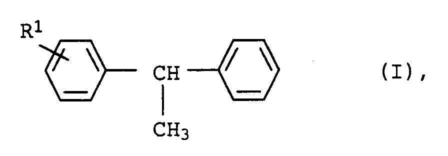 in der R¹ Wasserstoff oder C₁- bis C₈- Alkyl bedeuten, durch Umsetzung von Benzol mit Styrolen der allgemeinen Formel III 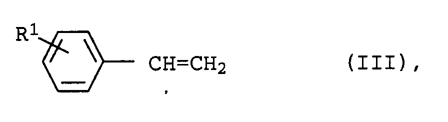 in der R¹ die oben genannten Bedeutungen hat, bei Temperaturen von 150 bis 350°C und Drücken von 5 bis 200 bar in Gegenwart von sauren Heterogenkatalysatoren durchführt, mit der Maßgabe, daß der Druck so hoch ist, daß unter den Reaktionsbedingungen das System in flüssiger oder überkritischer Phase bleibt, dadurch gekennzeichnet, daß man als saure Heterogenkatalysatoren großporige Zeolithe einsetzt.

2. Verfahren zur Herstellung von Diarylethanen I nach Anspruch 1, dadurch gekennzeichnet, daß man als Zeolithe Beta-Zeolithe, H-Y-Zeolithe oder Mordenite einsetzt.

3. Verfahren zur Herstellung von Diarylethanen I nach den Ansprüchen 1 bis 2, dadurch gekennzeichnet, daß R¹ Wasserstoff bedeutet.

4. Verfahren zur Herstellung von Diarylethanen I nach den Ansprüchen 1 bis 3, dadurch gekennzeichnet, daß man die Umsetzung bei Temperaturen von 180 bis 230°C durchführt.

5. Verfahren zur Herstellung von Diarylethanen I nach den Ansprüchen 1 bis 4, dadurch gekennzeichnet, daß man die Umsetzung unter Eigendruck oder durch zusätzliches Aufpressen von 1 bis 50 bar durchführt.

6. Verfahren zur Herstellung von Diarylethanen I nach den Ansprüchen 1 bis 5, dadurch gekennzeichnet, daß man die Umsetzung mit einer stationären Styrol-Konzentration von nicht mehr als 3% durchführt.

7. Verfahren zur Herstellung von Diarylethanen I nach den Ansprüchen 1 bis 5, dadurch gekennzeichnet, daß man die Umsetzung mit einer stationären Styrol-Konzentration von nicht mehr als 0,1% durchführt.

8. Verfahren zur Herstellung von Diarylethanen I nach den Ansprüchen 1 bis 5, dadurch gekennzeichnet, daß man die Umsetzung mit einer stationären Styrol-Konzentration von nicht mehr als 0,05% durchführt.

9. Verfahren zur Herstellung von Diarylethanen I nach den Ansprüchen 1 bis 8, dadurch gekennzeichnet, daß man die Umsetzung in einem kontinuierlich durchmischten Reaktor oder in einem Festbettreaktor durchführt.

10. verfahren zur Herstellung von Diarylethanen I nach den Ansprüchen 1 bis 9, dadurch gekennzeichnet, daß bei der Umsetzung in einem Festbettreaktor das Reaktionsgemisch unter starker Rückdurchmischung mehrmals durch dieses Festbett gefahren wird, wobei dem Kreisstrom kontinuierlich frische Eduktlösung zudosiert und eine äquivalente Menge Produktlösung kontinuierlich abgezogen wird.

## Claims

1. A process for the preparation of a diarylethane of the formula I 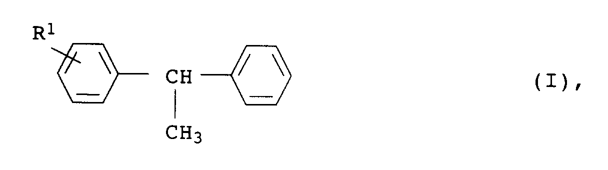 where R¹ is hydrogen or C₁-C₈- alkyl, by reacting benzene with the styrene of the formula III 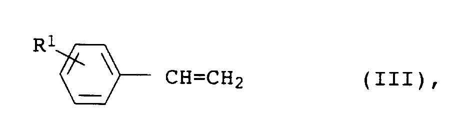 where R¹ has the abovementioned meanings, at from 150 to 350°C and from 5 to 200 bar in the presence of an acidic heterogeneous catalyst, with the proviso that the pressure is so high that the system remains in the liquid or supercritical phase under the reaction conditions, wherein the acidic heterogeneous catalyst used is a large-pore zeolite.

2. A process for the preparation of a diarylethane I as claimed in claim 1, wherein the zeolite used is a beta-zeolite, an HY zeolite or a mordenite.

3. A process for the preparation of a diarylethane I as claimed in claims 1 and 2, wherein R¹ is hydrogen.

4. A process for the preparation of a diarylethane I as claimed in any of claims 1 to 3, wherein the reaction is carried out at from 180 to 230 °C.

5. A process for the preparation of a diarylethane I as claimed in any of claims 1 to 4, wherein the reaction is carried out under autogenous pressure or by increasing the pressure by from 1 to 50 bar.

6. A process for the preparation of a diarylethane I as claimed in any of claims 1 to 5, wherein the reaction is carried out using a steady-state styrene concentration of not more than 3%.

7. A process for the preparation of a diarylethane I as claimed in any of claims 1 to 5, wherein the reaction is carried out using a steady-state styrene concentration of not more than 0.1%.

8. A process for the preparation of a diarylethane I as claimed in any of claims 1 to 5, wherein the reaction is carried out using a steady-state styrene concentration of not more than 0.05%.

9. A process for the preparation of a diarylethane I as claimed in any of claims 1 to 8, wherein the reaction is carried out in a continuously mixed reactor or in a fixed-bed reactor.

10. A process for the preparation of a diarylethane I as claimed in any of claims 1 to 9, wherein, in the case of the reaction in a fixed-bed reactor, the reaction mixture is passed several times through this fixed bed with vigorous backmixing, fresh solution of starting materials being metered continuously into the circulation and an equivalent amount of product solution being removed continuously.

## Revendications

1. Procédé de préparation de diaryléthanes de formule générale I 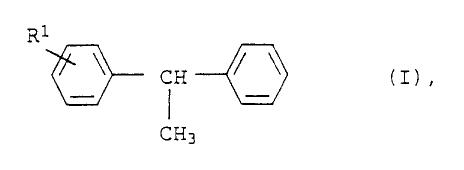 dans laquelle R¹ représente de l'hydrogène ou un alkyle en C₁ à C₈, par réaction du benzène avec des styrènes de formule générale III 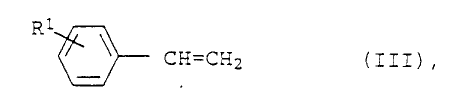 dans laquelle R¹ a les significations précitées, effectué à des températures de 150 à 350°C et sous une pression de 5 à 200 bar en présence de catalyseurs hétérogènes acides avec pour condition que la pression soit suffisamment élevée pour que le système reste en phase liquide ou supercritique dans les conditions de la réaction, caractérisé en ce que l'on utilise, comme catalyseur hétérogène acide, des zéolithes à grands pores.

2. Procédé de préparation de diaryléthanes 1 selon la revendication 1, caractérisé en ce que l'on utilise comme zéolithes des zéolithes bêta, des zéolithes H-Y ou des mordénites.

3. Procédé de préparation de diaryléthanes I selon les revendications 1 et 2, caractérisé en ce que R¹ représente de l'hydrogène.

4. Procédé de préparation de diaryléthanes I selon les revendications 1 à 3, caractérisé en ce que l'on effectue la réaction à des températures comprises entre 180 et 230°C.

5. Procédé de préparation de diaryléthanes I selon les revendications 1 à 4, caractérisé en ce que l'on effectue la réaction sous pression propre ou avec compression supplémentaire allant de 1 à 50 bar.

6. Procédé de préparation de diaryléthanes I selon les revendications 1 à 5, caractérisé en ce que l'on effectue la réaction avec une concentration fixe en styrène ne dépassant pas 3%.

7. Procédé de préparation de diaryléthanes I selon les revendications 1 à 5, caractérisé en ce que l'on effectue la réaction avec une concentration fixe en styrène ne dépassant pas 0,1%.

8. Procédé de préparation de diaryléthanes I selon les revendications 1 à 5, caractérisé en ce que l'on effectue la réaction avec une concentration fixe en styrène ne dépassant pas 0,05%.

9. Procédé de préparation de diaryléthanes I selon les revendications 1 à 8, caractérisé en ce que l'on effectue la réaction dans un réacteur à mélange continu ou dans un réacteur à lit fixe.

10. Procédé de préparation de diaryléthanes I selon les revendications 1 à 9, caractérisé en ce que, au cours de la réaction dans un réacteur à lit fixe, on fait passer le mélange réactionnel de nombreuses fois à travers ce lit fixe avec une forte remise en mélange, avec addition continue au débit en circulation de solutions fraîches d'éduct et avec soutirage en continu d'une quantité équivalente de solution de produit.
